# EUROPÄISCHE PATENTANMELDUNG

(11) **EP 4 741 367 A1**
(43) Veröffentlichungstag der Anmeldung: **13.05.2026**
(21) Anmeldenummer: 25210755.2
(22) Anmeldetag: 23.10.2025
(51) Int. Cl.: C07C 2/10, C07C 7/09, C07C 11/02, C07C 11/04, C07C 11/10, C07C 11/107

(54) **ANREICHERUNG VON ETHEN IN OLEFINGEMISCHEN MITTELS HETEROGEN-KATALYSIERTER, SELEKTIVER CO-OLIGOMERISIERUNG VON C3+-OLEFINEN**

(30) Priorität: 12.11.2024 DE 102024133043
(71) Anmelder: Karlsruher Institut für Technologie, 76131 Karlsruhe (DE)
(72) Erfinder: Fuchs, Constantin, 76275 Ettlingen (DE); Arnold, Dr. Ulrich, 76646 Bruchsal (DE); Sauer, Prof. Dr. Jörg, 76135 Karlsruhe (DE)

(57) **Zusammenfassung**

Die vorliegende Erfindung betrifft ein Verfahren zur Anreicherung von Ethen in Olefingemischen bei gleichzeitiger Co-Oligomerisierung von C₃₊-Olefinen in einem Festbettreaktor unter milden Reaktionsbedingungen mittels eines heterogenen amorphen Siliciumoxid-Aluminiumoxid-Katalysators, der eine milde Azidität aufweist.

## Beschreibung

Die vorliegende Erfindung betrifft ein Verfahren zur Anreicherung von Ethen in Olefingemischen mittels heterogenkatalysierter, selektiver Co-Oligomerisierung von C₃₊-Olefinen.

Kurzkettige Olefine im Kohlenstoffkettenlängenbereich von C₂₋₄ sind wichtige Grundbausteine der chemischen Industrie und weisen einen breiten und stetig wachsenden Anwendungsbereich auf. Beispielsweise sind Ethen und Propen für die Kunststoffindustrie unverzichtbar. Des Weiteren lassen sich aus Folgeprodukten wie z.B. Ethylenoxid Desinfektionsmittel, Dämmstoffe oder Tenside für Wasch- und Reinigungsmittel herstellen.

Großindustriell werden Olefine in Raffinerien durch Steamcracking von fossilem Naphtha erzeugt. Weiterhin lassen sich Olefine über Methanol bzw. Dimethylether (DME) über den Methanol-to-Olefins (MTO)- bzw. DME-to-Olefins (DTO)-Prozess herstellen.

Für die weitere Verarbeitung der Olefine muss das Olefingemisch aufgetrennt werden. Dafür wird gegenwärtig eine sehr komplexe kryogene Destillation eingesetzt. Der Ursprung des Olefingemischs, ob fossil oder aus erneuerbaren Quellen, spielt dabei keine Rolle. Die erforderlichen Prozessbedingungen für die destillative Trennung der Olefine beinhalten mehrere Prozessschritte mit variierenden Anforderungen an Temperatur und Druck. Zudem ist der materielle und apparative Aufwand mit bis zu 200 Trennböden pro benötigter Trennkolonne enorm, wodurch der gesamte Prozess mit hohen Investitions- und Betriebskosten verbunden ist.

In der EP0683146A1 wird die Separation von Ethen aus einem Olefingemisch mittels kryogener Destillation bei Temperaturen von bis zu -43 °C und Drücken von bis zu 36 bar durchgeführt. Diese Bedingungen werden durch mehrstufige Kompressionsvorgänge mit anschließender Abkühlung erreicht. Während höhere Olefine ab C₃ unter diesen Bedingungen kondensieren, bleibt Ethen gasförmig und kann folglich mittels Rektifikationskolonnen mit Kolonnenhöhen von bis zu 50 m abgetrennt werden. Dieser Prozess der Olefinseparation ist äußerst energieintensiv, insbesondere die Bereitstellung der benötigten Kälte und des Drucks.

Eine weitere Möglichkeit der Olefinabtrennung ist die Nutzung von adsorptiven Verfahren, z.B. mittels kristalliner Molsiebe oder metallorganischer Gerüstverbindungen. Wie beispielsweise in der US6200366B1 beschrieben, ist durch die gezielte Einstellung der Porendurchmesser von Zeolithen die Trennung unterschiedlich großer Moleküle möglich. In der US6517611B1 wird für die Separation von Ethen oder Propen von Paraffinen ein kristallines Titan-Silikat-Molsieb mit einstellbaren Porendurchmessern im Bereich von 0,3 bis 0,4 nm eingesetzt.

Adsorptive Verfahren weisen allerdings meist geringere Kapazitäten und Selektivitäten auf. Zudem sind die benötigten Druckwechsel apparativ und energetisch anspruchsvoll, wodurch hohe Kosten für die Anlagentechnik und den laufenden Anlagenbetrieb entstehen. Darüber hinaus kommt es im Dauerbetrieb zu einer reduzierten Trennwirkung der Adsorbentien, z.B. durch Blockade von Poren, was eine Regeneration der Materialien erforderlich macht.

Alternativ wird auf dem Gebiet der Membrantechnologie an Trennverfahren geforscht. Membranen finden in der Trennung von Olefinen und Paraffinen in Form von Transportmembranen (facilitated transport membrane - FTM) Anwendung, wobei der Transport der Olefine durch Trägermoleküle stattfindet, die mit den Olefinen reagieren. Ein solcher Prozess ist in der EP1552875A1 beschrieben. Membranen sind für eine großtechnische Anwendung dieser Art noch nicht ausreichend entwickelt. Ihre Herstellung ist aufwändig, da die zugrundeliegenden Materialien an die Erfordernisse angepasst werden müssen, z.B. durch Modifikation mit Metallen oder porösen Strukturelementen. Zudem kann es auch hier zu Verblockungen kommen, die zu einer verminderten Trennleistung führen. Um Stoffe mit hoher Reinheit abzutrennen, sind geringe Permeabilitäten nötig, was einen weiteren Schwachpunkt von Membranen darstellt. Dies konnte in der US5670051A durch die Einführung von metalldotierten Transportmembranen verbessert werden; allerdings verringerte sich dabei die Langzeitstabilität durch Deaktivierung der aktiven Komponenten (meist Silber oder Kupfer) auf der Membran, was zu Performanceverlusten führte.

Zuletzt sind noch absorptive Trennverfahren zu nennen. In der US4479812A wird ein recht aufwendiges absorptives Verfahren beschrieben, bei dem höhere Olefine ab C₃ in einer Absorptionskolonne mit einem großen Volumen ausgewaschen werden und ein ethenreicher Gasstrom bereitgestellt werden kann. Als Lösungsmittel kommen dabei höhere Kohlenwasserstoffe ab C₆ zum Einsatz. Das benötigte Gas-Flüssigkeit-Verhältnis limitiert hier den Durchsatz und wirkt sich auf die Dimension des Trennprozesses aus. Zudem sind die zulässigen Anteile an Ethen und höheren Olefinen im Eingangsstrom reglementiert. Bei absorptiven Verfahren müssen die Lösungsmittel regeneriert werden, z.B. durch Entgasung mittels Temperaturerhöhung oder Druckwechsel.

Eine energie- und kosteneffiziente Methode zur Ethenanreicherung und -abtrennung aus beliebigen Olefingemischen bei gleichzeitiger selektiver Co-Oligomerisierung von C₃₊-Olefinen ohne hohen apparativen Aufwand ist noch nicht beschrieben worden.

Somit liegt der vorliegenden Erfindung die Aufgabe zugrunde, ein Verfahren zur Anreicherung von Ethen in Olefingemischen mittels katalysierter, selektiver Co-Oligomerisierung von C₃₊-Olefinen bereitzustellen, wobei das Verfahren unter milden Reaktionsbedingungen ohne Einsatz aufwendiger Trennkolonnen durchgeführt werden soll und der eingesetzte Katalysator möglichst stabil und regenerierbar sein soll.

Die vorstehende technische Aufgabe wird durch die in den Ansprüchen gekennzeichneten Ausführungsformen gelöst.

Das zentrale Element der Erfindung ist die reaktive Anreicherung von Ethen in einem beliebigen Olefingemisch mittels selektiver Co-Oligomerisierung von Olefinen, d.h. der Verknüpfung leichter Olefine, vor allem von Propen und Buten, zu höheren Olefinen unter Einsatz eines heterogenen amorphen Siliciumoxid-Aluminiumoxid-Katalysators. Das Olefingemisch stammt bevorzugt aus einem Steamcracker oder einem MTO-Prozess bzw. DTO-Prozess und besteht hauptsächlich aus Ethen, Propen und Butenen. Höhere Olefine ab C₅ sind in einem solchen Gemisch nur in Spuren enthalten und wirken sich auf den erfindungsgemäßen Prozess nicht nachteilig aus. Aufgrund der für die Oligomerisierung eingesetzten, vergleichsweise niedrigen Temperaturen wird einzig Ethen an dem erfindungsgemäßen Oligomerisierungskatalysator nicht umgesetzt, wohingegen alle höheren Olefine ab Propen reagieren. Der Siliciumoxid-Aluminiumoxid-Katalysator hat keine Metallbeladung und zeichnet sich durch eine milde Azidität, eine amorphe Mesoporosität, eine hohe Langzeitstabilität und eine sehr gute Regenerierbarkeit aus. Höhere Olefine oligomerisieren bereitwillig bei Temperaturen um 120 °C an den Brønsted-Zentren dieses Katalysators, im Gegensatz zu Ethen, das erst ab 250 - 300 °C reagiert. Da alle Olefine außer Ethen reagieren, lässt sich Ethen im austretenden Gasstrom anreichern und anschließend effizient abtrennen, während die umgesetzten Olefine als Flüssigkeit separiert werden. Der Anteil an Ethen kann mit dem erfindungsgemäßen Verfahren im Falle eines Olefingemisches aus einem MTO-Prozess auf über 80 Gewichtsprozent gesteigert werden. Eine weitere Steigerung des Ethenanteils ist durch Reihenschaltung mehrerer Oligomerisierungsstufen möglich.

Die flüssigen Produkte der Co-Oligomerisierung weisen einen hohen Verzweigungsgrad auf und können somit zu Blendkomponenten für Benzin- oder Kerosinanwendungen weiterverarbeitet werden.

Das erfindungsgemäße Verfahren umfasst die folgenden Schritte:
A) Vorlegen eines kalzinierten amorphen Siliciumoxid-Aluminiumoxid-Katalysators mit einer spezifischen Oberfläche von 280 - 550 m²/g, einem Porenvolumen von 0,5 - 2 ml/g, einem Porendurchmesser von 5 - 15 nm, einer Partikelgröße von 250 bis 500 µm und einem SiO₂/Al₂O₃-Verhältnis im Bereich von 20:80 bis 70:30 Gewichtsprozent;
B) Inertisieren eines Festbettreaktors durch Ausheizen bei 250 - 350 °C und Durchströmen des Reaktors mit einem Inertgas;
C) Abkühlen des Reaktors auf 110 - 160 °C;
D) Einleiten eines gasförmigen oder flüssigen Olefingemisches in einen ersten Reaktorbereich des ausgeheizten Festbettreaktors unter Inertgas;
E) Gegebenenfalls Verdampfen des Olefingemisches aus Schritt D) im ersten Reaktorbereich;
F) Einleiten des Olefin-Gas-Gemisches in einen zweiten Reaktorbereich enthaltend ein Inertmaterial mit einer Partikelgröße von 250 - 500 µm und Vorwärmen des Olefin-Gas-Gemisches auf 110 - 160 °C;
G) Weiterleiten des Olefin-Gas-Gemisches in eine auf 110 - 160 °C aufgeheizte Reaktionszone enthaltend ein Gemisch aus dem Siliciumoxid-Aluminiumoxid-Katalysator und einem Inertmaterial mit einer Partikelgröße von jeweils 250 - 500 µm;
H) Einstellen eines Gesamtdrucks von 40 bar in der Reaktionszone mit einem Olefinpartialdruck von 28 - 38 bar und einem entsprechenden Inertgaspartialdruck von 2 - 12 bar;
I) Verweilen des Olefin-Gas-Gemisches in der Reaktionszone;
J) Entspannen der Reaktionsprodukte in einem dem Reaktor nachgeschalteten Anlagenbereich auf Umgebungsdruck, wobei die Oligomere mit Kettenlängen ab C₅ auskondensiert werden und die die angereicherte Ethenfraktion enthaltenden gasförmigen Olefine in der Gasphase abgetrennt werden.

Die Kalzinierung des amorphen Siliciumoxid-AluminiumoxidKatalysators erfolgt beispielsweise in einem Kalzinierungsofen, der mit 175 K/h aufgeheizt wird, bei einer Temperatur von 500 - 550 °C für 5 h. Die Inertisierung des Festbettreaktors in Schritt B) erfolgt beispielsweise für 8 - 12 h. Das im gesamten erfindungsgemäßen Verfahren verwendete Inertgas ist beispielsweise Argon oder Stickstoff.

In einer bevorzugten Ausführungsform wird in den Schritten C), F) und G) eine Temperatur von 120 °C eingestellt.

Das einzuleitende Edukt-Olefingemisch in Schritt D) ist beispielsweise ein Olefingemisch aus einem Methanol-to-Olefins (MTO)- bzw. DME-to-Olefins (DTO)-Prozess oder einem Steamcracking-Prozess und besteht hauptsächlich aus Ethen, Propen und Buten. In einer bevorzugten Ausführungsform liegen gemäß einer für den MTO-Prozess typischen Produktzusammensetzung im Edukt-Olefingemisch Ethen, Propen und Buten in der genannten Reihenfolge im molaren Verhältnis von 2:2:1 vor.

Das Inertmaterial in den Schritten F) und G) besteht aus Siliciumcarbid, Quarzglas, α-Aluminiumoxid oder einer technischen Keramik. Die Reaktionszone in Schritt G enthält den Siliciumoxid-Aluminiumoxid-Katalysator und das Inertmaterial mit einer Partikelgröße von jeweils 250 - 500 µm, wobei das Gemisch aus dem Siliciumoxid-Aluminiumoxid-Katalysator und dem Inertmaterial im Volumenverhältnis von Katalysator zu Inertmaterial von 0,1 - 0,2 vorliegt. In einer bestimmten Ausführungsform liegt der Siliciumoxid-Aluminiumoxid-Katalysator in Form von Pellets oder Extrudaten vor.

Die Verweilzeit des Olefingasgemisches in der Reaktionszone wird durch die gewichtsbezogene stündliche Raumgeschwindigkeit (WHSV) spezifiziert; diese wird im erfindungsgemäßen Verfahren zwischen 2 - 8 h⁻¹ eingestellt. Die WHSV ist definiert als das Gewicht des Einsatzmaterials, das pro Gewichtseinheit des Katalysators pro Stunde über den Katalysator fließt.

Liegen im Edukt-Olefingemisch Ethen, Propen und Buten in der genannten Reihenfolge im molaren Verhältnis von 2:2:1 vor und wird in Schritt H) ein Olefinpartialdruck von 36 bar und eine WHSV von 2 h⁻¹ eingestellt, so liegt nach dem erfindungsgemäßen Verfahren unter Einsatz eines einzigen Festbettreaktors der Anteil an Ethen im Produktgasgemisch bei mindestens 80 Gewichtsprozent (Tabelle 7).

In einer Ausführungsform des erfindungsgemäßen Verfahrens werden zwei funktionsgleiche Festbettreaktoren in Reihe geschaltet. Nach Abtrennung von Ethen wird das kurzkettige Produktgasgemisch vom ersten Festbettreaktor in den zweiten Festbettreaktor geleitet und das erfindungsgemäße Verfahren wird auch im zweiten Festbettreaktor durchgeführt, wobei im zweiten Festtbettreaktor der Olefinpartialdruck in Schritt H) auf 28.4 bar und der Inertgaspartialdruck auf 11.6 bar eingestellt wird. Durch das Hintereinanderschalten zweier funktionsgleicher Festbettreaktoren wird der angereicherte Ethenanteil im Produktgasgemisch um weitere 20 Gewichtsprozent erhöht (Tabellen 6 und 7).

In einer weiteren bestimmten Ausführungsform werden drei funktionsgleiche Festbettreaktoren in Reihe geschaltet und das erfindungsgemäße Verfahren wird auch in den nachgeschalteten Reaktoren durchgeführt. Nach der Abtrennung von Ethen wird das verbleibende kurzkettige Produktgasgemisch aus dem jeweils vorgeschalteten Festbettreaktor in den jeweils nachgeschalteten Festbettreaktor geleitet, wobei der Olefinpartialdruck im dem ersten Reaktor nachgeschalteten zweiten Reaktor in Schritt H) 28.4 bar und der Inertgaspartialdruck 11.6 bar beträgt und der Olefinpartialdruck in dem dritten in Reihe geschalteten Reaktor in Schritt H) auf 17.8 bar und der Inertgaspartialdruck auf 22.2 bar eingestellt wird.

Nach wiederholter Anwendung des erfindungsgemäßen Verfahrens ist eine weitere Anreicherung von Ethen bis zur gewünschten Endkonzentration beispielsweise mit Membrantrenntechniken möglich, wobei das erfindungsgemäße Verfahren für den Einsatz von nachgeschalteten Membrantrenntechniken einen großen Gewinn darstellt, da die eingesetzten Membranen dann einer deutlich geringeren Belastung ausgesetzt werden und nur noch geringe Gaskonzentrationen gehandhabt werden müssen.

In einer bestimmten Ausführungsform wird ein Teilstrom des Produktgasgemisches zur kontinuierlichen Bestimmung des Gehalts an gasförmigen Olefinen in einen online geschalteten Gaschromatographen (11) umgeleitet. Das mit Ethen angereicherte Produktgas wird nach der Abtrennung von der flüssigen Produktfraktion mit ad- bzw. absorptiven Verfahren, wie z.B. Molsieben oder Absorptionskolonnen, oder Membranverfahren behandelt. Somit wird nach der Abtrennung höherer Olefine eine größere Reinheit des Ethens erreicht. Aufgrund des geringen Anteils an höheren Olefinen im Produktgasgemisch wird mit dem erfindungsgemäßen Verfahren bei der Abtrennung von Ethen eine höhere Effizienz, Langlebigkeit und Wirtschaftlichkeit bei verringerter Trennleistung und Dimensionalität erreicht.

In einer bestimmten Ausführungsform wird ein Teil der flüssigen Produktphase zur kontinuierlichen Bestimmung des C₅₊-Olefingehalts (Oligomere mit Kettenlängen ab C₅) mit einem externen nachgeschalteten Gaschromatographen analysiert.

In der flüssigen Produktphase befinden sich C₅₊-Kohlenwasserstoffe mit einem hohen Verzweigungsgrad. Durch die Beeinflussung des Produktspektrums der Co-Oligomerisierung mittels variabler Reaktionsbedingungen ist es möglich, die Produktselektivität bezüglich der Olefinkettenlänge und des Verzweigungsgrades bis zu einem bestimmten Grad zu steuern.

In einer bestimmten Ausführungsform wird die flüssige Produktphase nach der Abtrennung von der gasförmigen Produktphase hydriert. Nach der Hydrierung der C₅₊-Olefine können diese als rein paraffinische Blendkomponenten für Benzin- oder Kerosingemische eingesetzt werden. Durch die Abwesenheit von Aromaten wird die Partikelbildung bei der motorischen Verbrennung dieser Kraftstoffe deutlich reduziert, was sich vorteilhaft auf das Emissionsverhalten auswirkt.

Der im erfindungsgemäßen Verfahren eingesetzte amorphe Silicium-Aluminiumoxid-Katalysator weist eine hohe Langzeitstabilität auf. Eine Regenerierung des Katalysators ist im Festbettreaktor unter Schutzgas durch eine Temperaturerhöhung auf 300 °C möglich. Dabei werden langkettige Oligomere von der Katalysatoroberfläche desorbiert, und der Katalysator weist anschließend wieder die ursprüngliche Aktivität hinsichtlich der Umwandlung von C₃₊-Olefinen auf. Sollte es zur Bildung von Koks auf dem Katalysator kommen, kann dieser zur Wiederherstellung der Aktivität mit Luftsauerstoff abgebrannt werden.

Das erfindungsgemäße Verfahren ist sehr energie- und kosteneffizient und ermöglicht in einem Festbettreaktor unter milden Reaktionsbedingungen die Co-Oligomerisierung von C₃₊-Olefinen und, bei gleichzeitiger Inertheit von Ethen, eine effiziente Anreicherung von Ethen. Das Verfahren lässt sich mit einem beliebigen Ethen-, Propen- und Buten-enthaltenden Olefingemisch und unabhängig von den jeweiligen Anteilen der Olefine in der Olefinmischung durchführen. Die Trennung der Produktgasphase von flüssigen Reaktionsprodukten ist apparativ mit geringem Aufwand ohne kryogene Rektifikationskolonnen durchführbar. Der eingesetzte Silicium-Aluminiumoxid-Katalysator hat keine Metallbeladung und liegt als Feststoff vor. Somit wird der Katalysator nicht aus dem Festbettreaktor ausgetragen, und es kommt nicht zur Mischung von Katalysatorkomponenten mit dem Produktgasgemisch oder der flüssigen Produktphase.

Mittels des hier beschriebenen Verfahrens ist es möglich, paraffinische Blendkomponenten für Benzin- und Kerosingemische mit einem günstigen Emissionsverhalten auf Basis verschiedener Ausgangsstoffe aus fossilen und/oder erneuerbaren Quellen herzustellen.

Die Erfindung wird anhand der folgenden Figuren, Ausführungsbeispiele und Beschreibungen näher erläutert.

Alle dargestellten Merkmale und deren Kombinationen sind nicht nur auf diese Figuren und Ausführungsbeispiele und deren Ausgestaltungen begrenzt. Vielmehr sollen diese stellvertretend für weitere mögliche, aber nicht explizit als Ausführungsbeispiele dargestellte weitere Ausgestaltungen kombinierbar angesehen werden.

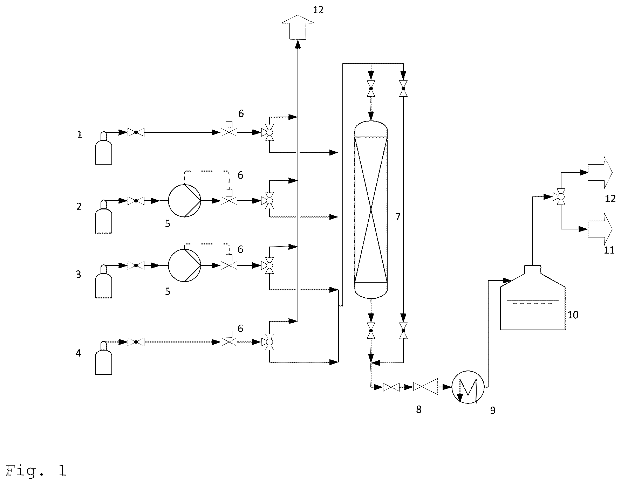

Figur 1 zeigt einen schematischen Aufbau einer Reaktoranlage zur Anreicherung von Ethen in Olefingemischen.

### Bezugszeichenliste

- 1: Ethen-Eduktgas
- 2: Propen-Eduktgas
- 3: 1-Buten / iso-Buten-Eduktgas
- 4: Argon-Inertgas
- 5: HPLC-Pumpe über Durchflussmesser geregelt
- 6: Durchflussmesser
- 7: Festbettreaktor
- 8: Druckhalteventil
- 9: Gegenstromkühler
- 10: Kondensat-Behälter
- 11: Online-Gaschromatograph
- 12: Abgas

### Beispiele

### Beispiel 1:

### Konditionierung der Katalysatoren:

Als Katalysatoren für die Co-Oligomerisierung werden in Beispiel 1 amorphe Silica-Alumina-Materialien mit dem Handelsnamen SIRALOX der Firma Sasol eingesetzt. Zum Einsatz kommen drei unterschiedliche Spezifikationen, SIRALOX 20, 40 und 70. Die Zahl gibt jeweils den Anteil an SiO₂ in Prozent an. Die Differenz zu 100 ergibt den Anteil an Al₂O₃. Die Materialien unterscheiden sich in ihren Eigenschaften wie der spezifischen Oberfläche, dem mittleren Porendurchmesser und der Anzahl an Lewis- und Brønsted-Säurezentren (Tabelle 1). Bevor die Katalysatoren zum Einsatz kommen, werden diese zunächst über einen Zeitraum von 5 h bei 550 °C kalziniert. Anschließend wird die Partikelgröße mittels Siebfraktionierung auf 250 bis 500 µm eingestellt.

**Tabelle 1: Eigenschaften der eingesetzten Katalysatoren**

| Eigenschaft | SIRALOX 20 | SIRALOX 40 | SIRALOX 70 |
|---|---|---|---|
| spezifische BET-Oberfläche [m²/g] | 495 | 455 | 331 |
| Porenvolumen [ml/g] | 1.26 | 1.57 | 1.44 |
| mittlerer Porendurchmesser [nm] | 7.3 | 9.2 | 11.2 |
| Brønsted-Zentren [µmol/g] | 36.88 | 70.98 | 49.73 |
| Lewis-Zentren [µmol/g] | 194.61 | 156.65 | 65.61 |

### Beispiel 2:

Ausführung der Laboranlage:
Die unterschiedlichen Olefine werden aus separaten Gasflaschen entnommen und dem Rohrreaktor zugeführt. Ethen wird gasförmig eingeleitet, höhere Olefine, wie Propen und 1-Buten, werden flüssig mittels Pumpen in den Reaktor gefördert und verdampfen dort in der Einlaufstrecke. Dort erfolgt auch die Vermischung der Komponenten und es bildet sich ein kolbenförmiges Strömungsprofil aus. Dieser erste Bereich des Reaktors ist mit Silicium-Carbid (SiC)-Partikeln (d_{SiC} = 250 - 500 µm) gefüllt und dient auch zur Vorwärmung der Edukte. In der sich anschließenden Reaktionszone befindet sich ein Gemisch aus Katalysator und SiC, um eine isotherme Reaktionsführung der exothermen Oligomerisierung zu gewährleisten. Das Verhältnis von Katalysator zu SiC liegt bei 1:10 und die Partikelgrößen liegen jeweils im Bereich von 250 - 500 µm. Nach der Reaktionszone ist der Reaktor wieder ausschließlich mit SiC-Partikeln gefüllt. Ein Fließbild der Anlage ist in Figur 1 dargestellt.

### Beispiel 3:

Allgemeine Versuchsdurchführung und Analytik:
Der Reaktor wird über einen Zeitraum von 12 h bei 300 °C und unter einem Argonstrom ausgeheizt, um Wasser und andere flüchtige Bestandteile auszutragen. Anschließend wird der Reaktor auf die Reaktionstemperatur von 120 °C abgekühlt und der Reaktordruck wird mit Argon aufgebaut. Danach werden die Olefine Ethen, Propen und 1-Buten zudosiert, wobei eine für den MTO-Prozess typische Produktzusammensetzung gewählt wird (40 mol% Ethen, 40 mol% Propen und 20 mol% 1-Buten). Die Regelung erfolgt über Mass Flow Controller, welche einen über die Versuchsdauer gleichbleibenden Massenstrom der einzelnen Olefine garantieren. Nach Durchlaufen der Reaktionszone erfolgt die Entspannung der Produkte auf Umgebungsdruck, wobei die Oligomere mit Kettenlängen ab C₅ auskondensiert werden. Gasförmige Olefine werden online in einem Gaschromatographen (HP 5890 mit Rt-Alumina BOND/Na₂SO₄-Säule) analysiert, die flüssigen Produkte werden gesammelt, gewogen und nach Beendigung des Versuchs mittels offline-Gaschromatographie (Agilent 6890 mit DB-1-Säule) identifiziert und quantifiziert.

### Beispiel 4:

Anreicherung von Ethen in einem Ethen/Propen/1-Buten-Gemisch: In diesem Beispiel erfolgte die Anreicherung von Ethen in einem Gemisch aus Ethen (40 mol%, 28.6 Gew.%), Propen (40 mol%, 42.8 Gew.%) und 1-Buten (20 mol%, 28.6 Gew.%). Die dabei eingesetzten Katalysatoren, die Laboranlage und die allgemeine Vorgehensweise sind in den Beispielen 1 bis 3 beschrieben. Es wurden 6 verschiedene Oligomerisierungsexperimente mit variablen Prozessparametern durchgeführt. Die jeweiligen Prozessparameter sind in Tabelle 2 dargestellt. Die mit den Katalysatoren SIRALOX 20, 40 und 70 bei 32 bar Olefinpartialdruck (40 bar Gesamtdruck mit Argon) und einer WHSV von 4 h⁻¹ (Experimente 1-3) erzielten Umsätze der einzelnen Olefine sind in Tabelle 3 zusammengefasst. Die entsprechenden Ergebnisse für den Katalysator SIRALOX 40 bei einer höheren WHSV von 8 h⁻¹ sind ebenfalls enthalten (Experiment 4).

In einem weiteren Experiment wurden in einer Simulation die nicht umgesetzten Olefine des vorangegangenen Experiments 4 als Einsatzstoffe in einem zweiten, dem ersten Festbettreaktor nachgeschalteten Festbettreaktor verwendet. Hierbei entsprachen die Reaktionsbedingungen im zweiten Festbettreaktor denen des ersten Festbettreaktors, allerdings wurde der Olefinpartialdruck auf 28.4 bar reduziert (siehe Experiment 5 in den jeweiligen Tabellen). Wie aus Tabelle 3 ersichtlich ist, wurde auch in diesem Experiment kein Ethen umgesetzt. Es verhält sich somit unter den gegebenen Reaktionsbedingungen inert, wohingegen Propen und 1-Buten erneut reagieren und flüssige Oligomere bilden. Der Gesamtumsatz an Propen und 1-Buten kann somit durch eine Reihenschaltung von mehreren Reaktoren erhöht werden.

Die höchsten Umsätze an Propen und 1-Buten wurden bei einer WHSV von 2 h⁻¹ sowie einem verringerten Argonanteil von 10 % erzielt (Experiment 6). Der Gesamtdruck blieb bei 40 bar, der Olefinpartialdruck stieg von 32 auf 36 bar. Unter diesen Bedingungen wurde folglich die beste Anreicherung von Ethen in der Gasphase erzielt. Im Produktgas konnte somit der Massenanteil an Ethen von 28.6 Gew.% auf 81.4 Gew.% erhöht werden. Wie bei einer WHSV von 8 h⁻¹ gezeigt wurde, kann durch die Reihenschaltung eines zweiten Reaktors die Umsetzung der höheren Olefine nochmals verbessert werden und somit die Effizienz der Ethenanreicherung im Produktgas weiter erhöht werden.

In Tabelle 4 sind die Selektivitäten der gebildeten flüssigen Kohlenwasserstoffe zu Benzin (C₅₋₁₀) - und Kerosin (C₉₋₁₆) - Komponenten angegeben. Die Produktverteilungen für Produkte mit Kettenlängen ab C₅ sind in Tabelle 5 zusammengefasst.

**Tabelle 2: Prozessparameter der Oligomerisierungen im Falle eines Ethen/Propen/1-Buten-Gemisches**

| Experiment | 1 | 2 | 3 | 4 | 5 | 6 |
|---|---|---|---|---|---|---|
| Katalysator | Siralox 20 | Siralox 40 | Siralox 70 | Siralox 40 | Siralox 40 | Siralox 40 |
| WHSV [h⁻¹] | 4 | 4 | 4 | 8 | 8 | 2 |
| Gesamtdruck [bar] | 40 | 40 | 40 | 40 | 40 | 40 |
| Olefinpartialdruck [bar] | 32 | 32 | 32 | 32 | 28.4 | 36 |
| Argonanteil [mol%] | 20 | 20 | 20 | 20 | 29 | 10 |

**Tabelle 3: Olefin-Umsätze für unterschiedliche Reaktionsbedingungen im Falle eines Ethen/Propen/1-Buten-Gemisches**

| Experiment | 1 | 2 | 3 | 4 | 5 | 6 |
|---|---|---|---|---|---|---|
| X_{C2} [%] | 0 | 0 | 0 | 0 | 0 | 0 |
| X_{C3} [%] | 38.68 | 63.99 | 51.21 | 43.52 | 23.84 | 89.6 |
| X_{C4} [%] | 40.53 | 64.84 | 52.54 | 46.44 | 20.34 | 91.3 |

**Tabelle 4: Benzin (C₅₋₁₀) - und Kerosin (C₉₋₁₆)-Selektivitäten für unterschiedliche Reaktionsbedingungen im Fall eines Ethen/Propen/1-Buten-Gemisches**

| Experiment | 1 | 2 | 3 | 4 | 5 | 6 |
|---|---|---|---|---|---|---|
| Benzin (C₅₋₁₀) - Selektivität [Gew.%] | 55.54 | 42.00 | 49.19 | 50.72 | 44.75 | 30.23 |
| Kerosin (C₉₋₁₆) - Selektivität [Gew.%] | 77.95 | 85.51 | 86.64 | 80.35 | 91.16 | 90.44 |

**Tabelle 5: Zusammensetzung der flüssigen Oligomerisierungsprodukte für unterschiedliche Reaktionsbedingungen im Falle eines Ethen/Propen/1-Buten-Gemisches**

| Experiment | 1 | 2 | 3 | 4 | 5 | 6 |
|---|---|---|---|---|---|---|
| C₅^{a} | 0.40 | 0.73 | 0.01 | 0.65 | 0.00 | 0.16 |
| C₆ | 1.38 | 1.10 | 0.31 | 1.44 | 0.04 | 0.46 |
| C₇ | 8.66 | 5.00 | 4.04 | 7.76 | 1. 64 | 2.77 |
| C₈ | 11.59 | 7.64 | 8.98 | 9.78 | 7.15 | 6.16 |
| C₉ | 17.04 | 13.31 | 16.75 | 14.81 | 17.29 | 10.57 |
| C₁₀ | 16.44 | 14.18 | 19.08 | 16.25 | 18.61 | 10.11 |
| C₁₁ | 10.90 | 11.36 | 12.27 | 10.55 | 12.73 | 10.05 |
| C₁₂ | 13.77 | 16.86 | 16.22 | 14.49 | 14.76 | 16.16 |
| C13+ | 19.77 | 29.77 | 22.30 | 24.23 | 27.75 | 43.55 |

| | | | | | | |
|---|---|---|---|---|---|---|
| ^{a}Angaben in Gew.% | | | | | | |

**Tabelle 6: Anteil des Ethens im Produktgas (inklusive Argon)**

| Experiment | 1 | 2 | 3 | 4 | 5 | 6 |
|---|---|---|---|---|---|---|
| Molanteil C₂H₄ | 39.2 | 46.3 | 42.6 | 42.0 | 50.3 | 71.1 |
| Massenanteil C₂H₄ | 29.4 | 35.9 | 32.3 | 31.7 | 40.0 | 61.2 |

**Tabelle 7: Anteil des Ethens im Olefinproduktgas (exklusive Argon)**

| Experiment | 1 | 2 | 3 | 4 | 5 | 6 |
|---|---|---|---|---|---|---|
| Molanteil C₂H₄ | 52.4 | 65.1 | 58.0 | 55.9 | 71.7 | 87.8 |
| Massenanteil C₂H₄ | 39.8 | 52.8 | 45.3 | 43.3 | 60.3 | 81.4 |

## Patentansprüche

1. Verfahren zur Anreicherung von Ethen und zur selektiven Co-Oligomerisierung von C₃₊-Olefinen in Olefingemischen umfassend die folgenden Schritte:
A) Vorlegen eines kalzinierten amorphen Siliciumoxid-Aluminiumoxid-Katalysators mit einer spezifischen Oberfläche von 280 - 550 m²/g, einem Porenvolumen von 0,5 - 2 ml/g, einem Porendurchmesser von 5 - 15 nm, einer Partikelgröße von 250 bis 500 µm und einem SiO₂/Al₂O₃-Verhältnis im Bereich von 20:80 bis 70:30 Gewichtsprozent;
B) Inertisieren eines Festbettreaktors durch Ausheizen bei 250 - 350 °C und Durchströmen des Reaktors mit einem Inertgas;
C) Abkühlen des Reaktors auf 110 - 160 °C;
D) Einleiten eines gasförmigen oder flüssigen Olefingemisches in einen ersten Reaktorbereich des ausgeheizten Festbettreaktors unter Inertgas;
E) Gegebenenfalls Verdampfen des Olefingemisches aus Schritt D) im ersten Reaktorbereich;
F) Einleiten des Olefin-Gas-Gemisches in einen zweiten Reaktorbereich enthaltend ein Inertmaterial mit einer Partikelgröße von 250 - 500 µm und Vorwärmen des Olefin-Gas-Gemisches auf 110 - 160 °C;
G) Weiterleiten des Olefin-Gas-Gemisches in eine auf 110 - 160 °C aufgeheizte Reaktionszone enthaltend ein Gemisch aus dem Siliciumoxid-Aluminiumoxid-Katalysator aus Schritt A) und einem Inertmaterial mit einer Partikelgröße von 250 - 500 µm;
H) Einstellen eines Gesamtdrucks von 40 bar in der Reaktionszone mit einem Olefinpartialdruck von 28 - 38 bar und einem entsprechenden Inertgaspartialdruck von 2 - 12 bar;
I) Verweilen des Olefin-Gas-Gemisches in der Reaktionszone;
J) Entspannen der Reaktionsprodukte in einem dem Reaktor nachgeschalteten Anlagenbereich auf Umgebungsdruck, wobei die Oligomere mit Kettenlängen ab C₅ auskondensiert werden und die die angereicherte Ethenfraktion enthaltenden gasförmigen Olefine in der Gasphase abgetrennt werden.

2. Verfahren nach Anspruch 1, wobei zwei funktionsgleiche Festbettreaktoren in Reihe geschaltet werden und das Verfahren gemäß Anspruch 1 auch im dem ersten Festbettreaktor nachgeschalteten zweiten Festbettteaktor durchgeführt wird, wobei das kurzkettige Produktgasgemisch aus dem ersten Festbettreaktor nach der Abtrennung von Ethen in den zweiten Festbettteaktor geleitet wird und der Olefinpartialdruck im zweiten Festbettteaktor in Schritt H) auf 28.4 bar und der Inertgaspartialdruck auf 11.6 bar eingestellt wird.

3. Verfahren nach Anspruch 1, wobei drei funktionsgleiche Festbettreaktoren in Reihe geschaltet werden und das Verfahren gemäß Anspruch 1 auch in den dem ersten Festbettreaktor nachgeschalteten Festbettreaktoren durchgeführt wird, wobei das kurzkettige Produktgasgemisch aus dem jeweils vorgeschalteten Festbettreaktor nach der Abtrennung von Ethen in den nachfolgend geschalteten Festbettreaktor geleitet wird und der Olefinpartialdruck im dem ersten Reaktor nachgeschalteten zweiten Festbettreaktor in Schritt H) 28.4 bar und der Inertgaspartialdruck 11.6 bar beträgt und der Olefinpartialdruck in dem dem zweiten Festbettreaktor nachgeschalteten Festbettreaktor in Schritt H) auf 17.8 bar und der Inertgaspartialdruck auf 22.2 bar eingestellt wird.

4. Verfahren nach einem der vorangehenden Ansprüche, wobei das Inertmaterial in den Schritten F) und G) Siliciumcarbid, Quarzglas, α-Aluminiumoxid oder eine technische Keramik ist.

5. Verfahren nach einem der vorangehenden Ansprüche, wobei das Gemisch aus dem Siliciumoxid-Aluminiumoxid-Katalysator und dem Inertmaterial in Schritt G) im Volumenverhältnis von Katalysator zu Inertmaterial von 0,1 - 0,2 vorliegt.

6. Verfahren nach einem der vorangehenden Ansprüche, wobei der Katalysator in Form von Pellets oder Extrudaten vorliegt.

7. Verfahren nach einem der vorangehenden Ansprüche, wobei in den Schritten C), F) und G) eine Temperatur von 120 °C eingestellt wird.

8. Verfahren nach einem der vorangehenden Ansprüche, wobei im Reaktor eine gewichtsbezogene stündliche Raumgeschwindigkeit (WHSV) von 2 - 8 h⁻¹ eingestellt wird.

9. Verfahren nach einem der vorangehenden Ansprüche, wobei im Edukt-Olefingemisch Ethen, Propen und Buten in der genannten Reihenfolge im molaren Verhältnis von 2:2:1 vorliegen.

10. Verfahren nach Anspruch 9, wobei die WHSV 2 h⁻¹ und der Olefinpartialdruck in Schritt H) 36 bar betragen.

11. Verfahren nach einem der vorangehenden Ansprüche, wobei zumindest ein Teilstrom des Produktgasgemisches zur kontinuierlichen Bestimmung des Gehalts an gasförmigen Olefinen in einen online geschalteten Gaschromatographen (11) umgeleitet wird.

12. Verfahren nach einem der vorangehenden Ansprüche, **dadurch gekennzeichnet, dass** mindestens ein Teil der flüssigen Produktphase zur kontinuierlichen Bestimmung des C₅₊-Olefingehalts (Oligomere mit Kettenlängen ab C₅) mit einem externen nachgeschalteten Gaschromatographen analysiert wird.

13. Verfahren nach einem der vorangehenden Ansprüche, wobei das mit Ethen angereicherte Produktgas nach der Abtrennung an Molsieben, Membranen oder mittels Absorptionskolonnen aufgereinigt wird.

14. Verfahren nach einem der vorangehenden Ansprüche, wobei die flüssige Produktphase nach der Abtrennung vom Produktgasgemisch hydriert wird.

15. Verwendung von nach dem Verfahren gemäß Anspruch 14 hergestellten paraffinischen hochverzweigten Kohlenwasserstoffen für die Herstellung von Blendkomponenten in Benzin- oder Kerosingemischen.
